# EUROPÄISCHE PATENTSCHRIFT

(11) **EP 3 184 002 B1**
(45) Veröffentlichungstag und Bekanntmachung des Hinweises auf die Patenterteilung: **20.02.2019**
(21) Anmeldenummer: 16196339.2
(22) Anmeldetag: 28.10.2016
(51) Int. Cl.: A47C 31/12, A61B 5/107

(54) **PERSONENVERMESSUNGSSTATION UND MESSVERFAHREN**
PERSON MEASURING STATION AND MEASURING METHOD
STATION DE MESURE DE PERSONNES ET PROCÉDÉ DE MESURE

(30) Priorität: 22.12.2015 DE 102015226521
(43) Veröffentlichungstag der Anmeldung: 28.06.2017
(73) Patentinhaber: Rössle & Wanner GmbH, 72116 Mössingen (DE)
(72) Erfinder: Greiner, Manfred, 72116 Mössingen (DE)
(74) Vertreter: BRP Renaud & Partner mbB Rechtsanwälte Patentanwälte Steuerberater

(56) Entgegenhaltungen:
- WO-A1-2013/079118
- AT-U1- 5 871
- DE-A1-102006 008 598
- DE-U1-202013 104 953
- JP-A- 2002 188 911
- Auburn Creative Ltd: "Leica DISTO S910 | Leica S910 Laser Distance Meter | Leica Laser Measure", , 6 April 2015 (2015-04-06), XP055462867, Retrieved from the Internet: URL:https://web.archive.org/web/2015040600 5826/http://www.laser-measure.co.uk/leica- disto/leica-disto-s910.html [retrieved on 2018-03-26]

## Beschreibung

Die vorliegende Erfindung betrifft eine Personenvermessungsstation zum Vermessen einer Seitenkontur einer Person zum Einstellen eines Liegesystems eines Betts. Die Erfindung betrifft ein Verfahren zum Vermessen einer Seitenkontur einer Person zum Einstellen eines Liegesystems eines Betts, insbesondere mittels einer derartigen Personenvermessungsstation.

WO 2013/079118 A1 zeigt eine Personenvermessungsstation nach dem Oberbegriff des Anspruchs 1. Ein Bett umfasst üblicherweise ein Gestell zur Aufnahme eines Liegesystems. Ein solches Liegesystem umfasst in der Regel zumindest eine vom Gestell zu tragende Unterfederung und eine auf der Unterfederung aufliegende Matratze. Die Unterfederung kann ein Lattenrost, wie z.B. ein Leistenlattenrost und ein Tellerlattenrost, oder ein Boxspringrost oder dergleichen sein. Für den Schlafkomfort einer Person kommt es neben der Auswahl einer geeigneten Matratze auch wesentlich auf die Unterfederung und die Einstellung der Unterfederung an. Ein optimaler Schlafkomfort ergibt sich, wenn das Liegesystem bei einem Seitenschläfer, also bei einer Person, die vorwiegend auf ihrer Seite schläft, an die Seitenkontur der Person angepasst ist. Durch die Anpassung des Liegesystems an die Seitenkontur der jeweiligen Person lässt sich insbesondere erreichen, dass in der Seitenlage die Wirbelsäule der Person weitgehend in einer, vorzugsweise horizontalen, Ebene liegt, so dass sich der Rücken der Person während des Schlafs entspannen und erholen kann.

Da die Physionomie der Menschen verschieden ist, muss für einen optimalen Liegekomfort das Liegesystem an die jeweilige Person angepasst werden. Dies erfolgt im Rahmen einer Einstellung des Liegesystems.

Im guten Fachhandel kann ein geübter Berater an der auf der Matratze liegenden Person erkennen, ob das Liegesystem gut eingestellt ist, also ob die Matratze richtig ausgewählt ist und die Unterfederung gut eingestellt ist oder ob eine Einstellung der Unterfederung verändert werden muss und/oder ob eine andere Matratze gewählt werden muss, um die Liegeposition zu verbessern. Bei einer solchen händischen Einstellung des Liegesystems handelt es sich häufig um einen langwierigen Vorgang, der mehrere Iterationsschritte benötigt, um eine zufriedenstellende Anpassung des Liegesystems an die jeweilige Person zu erhalten. Die Qualität dieser Anpassung des Liegesystems hängt jedoch stark von der Erfahrung und dem Geschick des Beraters ab.

Die vorliegende Erfindung beschäftigt sich mit dem Problem, für das Einstellen eines Liegesystems eines Betts an eine Person einen Weg aufzuzeigen, der die Qualität der Anpassung des Liegesystems an die jeweilige Person verbessert.

Dieses Problem wird erfindungsgemäß durch eine Personenvermessungsstation gemäß Anspruch 1 bzw. durch ein Messverfahren gemäß Anspruch 22 gelöst. Vorteilhafte Ausführungsformen sind Gegenstand der abhängigen Ansprüche.

Die Erfindung beruht hinsichtlich der Vorrichtung auf dem allgemeinen Gedanken, eine Personenvermessungsstation bereitzustellen, die einen Messraum aufweist, der nach hinten mit einer vertikalen Rückwand und seitlich mit wenigstens einer vertikalen Seitenwand begrenzt ist. Desweiteren ist der Messraum nach unten durch eine horizontale Standfläche begrenzt, auf der sich eine Person in einer Messposition hinstellen kann. Die Person, deren Seitenkontur vermessen werden soll, nimmt in der Messposition eine stehende Position ein, in der ein Rücken der Person der Rückwand der Personenvermessungsstation zugewandt oder davon abgewandt ist, während eine Seite der Person der genannten Seitenwand der Personenvermessungsstation zugewandt ist. In dieser Messposition kann nun mit Hilfe eines Abstandsmessgeräts der Personenvermessungsstation an mehreren vorbestimmten Bereichen der Seite der Person jeweils ein Abstand von der Seite der Person zu der Seitenwand gemessen werden, die dieser Seite der Person zugewandt ist. Die Personenvermessungsstation ist außerdem mit einer Auswerteeinheit ausgestattet, die mit dem Abstandsmessgerät zur Signalübertragung gekoppelt ist.

Die Erfindung beruht hierbei auf dem allgemeinen Gedanken, eine Seitenkontur der jeweiligen Person zu vermessen, um dann in Abhängigkeit der ermittelten Seitenkontur das Liegesystem und insbesondere die Unterfederung des Betts einstellen zu können. Die Vermessung der Seitenkontur soll dabei anhand vorbestimmter objektiver Kriterien durchgeführt werden, um subjektive Einflüsse einer Hilfsperson, welche die Messung durchführt, weitgehend auszuschließen.

Erfindungsgemäß bildet die Personenvermessungsstation eine Einheit zur Anordnung in einem Raum oder Zimmer eines Gebäudes. Die Personenvermessungsstation ist dann separat zu diesem Zimmer oder Raum des Gebäudes vorgesehen, in dem sie angeordnet ist. In der Folge sind die Wände der Personenvermessungsstation zusätzlich zu Wänden des Gebäudes vorhanden, die das jeweilige Zimmer oder den jeweiligen Raum seitlich begrenzen, in dem die Personenvermessungsstation angeordnet ist. Außerdem definiert dann die Personenvermessungsstation den Messraum als begrenzten Teil des Zimmers oder Raums, in dem die Personenvermessungsstation angeordnet ist. Durch diese separate Bauweise bzw. Anordnung der Personenvermessungsstation sind deren Wände definiert, was die Qualität der Vermessung erhöht.

Besonders vorteilhaft ist eine Ausführungsform, bei der die Auswerteeinheit so ausgestaltet bzw. programmiert ist, dass sie zum Vermessen der seitlichen Kontur der Person mehrere Bereiche der Seite der Person vorgibt, in denen jeweils der Abstand zur Seitenwand gemessen werden soll. Diese Abstandsmessungen werden dann nacheinander mit Hilfe der Auswerteeinheit durchgeführt, bspw. von einer Hilfsperson. Das hierbei verwendete Abstandsmessgerät kann zweckmäßig so ausgestaltet bzw. programmiert sein, dass es für den jeweiligen Bereich den gemessenen Abstand automatisch an die Auswerteeinheit übermittelt. Auf diese Weise können Übertragungsfehler ausgeschlossen werden. Schließlich kann bei einer besonders vorteilhaften Ausführungsform vorgesehen sein, dass die Auswerteeinheit so ausgestaltet bzw. programmiert ist, dass sie anhand der den einzelnen Bereichen zugeordneten Abstände die gewünschte Seitenkontur der jeweiligen Person ermittelt. Die so ermittelte Seitenkontur kann nun zum Einstellen des Liegesystems, insbesondere der Unterfederung, genutzt werden.

Die Auswerteeinheit kann zumindest einen Mikroprozessor aufweisen und speziell für die Personenvermessungsstation konfiguriert sein. Im einfachsten Fall kann die Auswerteeinheit durch einen entsprechend programmierten Computer gebildet sein.

Damit die jeweilige Hilfsperson die Abstandsmessungen möglichst einfach durchführen kann, ist das Abstandsmessgerät zweckmäßig so ausgestaltet, dass es einhändig bedienbar ist und/oder einhändig tragbar ist. Mit anderen Worten, das Abstandsmessgerät ist zweckmäßig vergleichsweise klein und vergleichsweise leicht, so dass es einfach und mühelos handhabbar ist. Zusammengefasst bedeutet dies, dass das Abstandsmessgerät zweckmäßig als Handheld ausgestaltet ist.

Erfindungsgemäß weist das Abstandsmessgerät ein hinteres Ende und ein vorderes Ende auf, wobei das Abstandsmessgerät zum Messen des jeweiligen Abstands an dem hinteren Ende mit der Seite der Person in Kontakt gebracht wird und an dem vorderen Ende den Abstand berührungslos misst. Hinteres und vorderes Ende sind bezüglich einer Messrichtung, in welcher der jeweilige Abstand gemessen wird, am Abstandsmessgerät voneinander abgewandt. Durch die Verwendung eines derartigen Abstandsmessgeräts, das mit seinem hinteren Ende mit der Person unmittelbar in Kontakt gebracht werden kann, ist es insbesondere möglich, die Seitenkontur der Person zu vermessen, während die Person angezogen ist. Störkonturen durch Kleidungsstücke lassen sich durch Andrücken des hinteren Endes des Abstandsmessgeräts der Kleidung an die Seite der Person eliminieren. Mit anderen Worten, die Kleidung der zu vermessenden Person wird im vorliegenden Zusammenhang zur Person hinzugezählt. Ein Hautkontakt ist nicht erforderlich. Die Möglichkeit, die Personen bekleidet zu vermessen, reduziert Hemmungen, den Vermessungsvorgang durchzuführen. Somit erleichtert sich diesbezüglich die Beratung und letztlich die Anpassung des- Liegesystems.

Erfindungsgemäß wird am vorderen Ende des Abstandsmessgeräts zum Messen des Abstands ein Messstrahl ausgestrahlt, der auf die Seitenwand auftrifft. Somit enthält das Abstandsmessgerät einen Sender für den Messstrahl. Desweiteren weist das Abstandsmessgerät zweckmäßig eine Messeinheit auf, die so ausgestaltet ist, dass sie eine am Abstandsmessgerät ankommende Reflexion des Messstrahls detektiert und die Zeit misst, die der Messstrahl vom Abstandsmessgerät zur Seitenwand und zurück benötigt. Anhand dieser Zeit kann diese Messeinheit den Abstand vom hinteren Ende des Abstandsmessgeräts bis zur Seitenwand berechnen. Mit anderen Worten, die Messeinheit beinhaltet einen Empfänger für den reflektierten Messstrahl sowie einen Rechner, um die Abstandsmessung anhand der Laufzeit des Messstrahls durchzuführen. Beispielsweise kann das Abstandsmessgerät auf Ultraschall-Technologie oder auf Laser-Technologie basieren, so dass der Messstrahl entweder ein Ultraschallstrahl oder ein Laserstrahl sein kann.

Ferner kann das Abstandsmessgerät drahtlost mit der Auswerteeinheit kommunizieren. Hierdurch vereinfacht sich die Handhabung des Abstandsmessgeräts. Eine weitere Vereinfachung der Handhabung ergibt sich, wenn in das Abstandsmessgerät außerdem einen Laserpointer im sichtbaren Lichtbereich integriert ist, der konzentrisch zum Messstrahl vom vorderen Ende abstrahlt. Somit sieht die Hilfsperson immer, gegenüber was der Abstand von der Seite der Person aktuell gemessen wird. Bei der Vermessung der Seitenkontur ist der Abstand zur jeweiligen Seitenwand zu messen, also sollte der Laserpointer auf diese Seitenwand zeigen.

Bei einer vorteilhaften Ausführungsform kann die jeweilige Seitenwand eine Messzone aufweisen, die sich an einem der Rückwand zugewandten hinteren Ende der Seitenwand befindet. Die Messzone zeichnet sich durch eine dem Messraum zugewandte Oberfläche aus, die in einer vertikalen Seitenebene liegt. Dabei erstreckt sich diese Seitenebene senkrecht zu einer vertikalen Rückseitenebene, in der eine dem Messraum zugewandte Oberfläche der Rückwand liegt. Mit Hilfe dieser Messzone vereinfacht sich die Abstandsmessung mit Hilfe eines Messstrahls, der an der Messzone reflektiert wird.

Bei einer Weiterbildung kann die jeweilige Seitenwand eine Konvergenzzone aufweisen, die nach vorn an die Messzone angrenzt und deren dem Messraum zugewandte Oberfläche in einer vertikalen Konvergenzebene liegt, die sich hinter der Rückwand mit einer vertikalen Mittelebene schneidet, die sich mittig durch die Rückwand und senkrecht zur Rückseitenebene erstreckt. Durch die Neigung der Konvergenzzone gegenüber der Messzone ist sichergestellt, dass die Abstandsmessung nicht in der Konvergenzzone durchgeführt werden kann, sondern nur im Bereich der Messzone erfolgt, deren Abstand zur Mittelebene bekannt ist. Dadurch ergibt sich eine relativ hohe Messgenauigkeit für die Abstandsmessungen. Ferner wird durch die Konvergenzzone erreicht, dass sich der Messraum nach vorn öffnet bzw. aufweitet und den Zugang zur Standfläche für die zu vermessende Person erleichtert. Ein Neigungswinkel zwischen der Messzone und der Konvergenzzone kann beispielsweise in einem Winkelbereich von 20° bis 70° oder von 20° bis 45° liegen. Bevorzugt ist ein Neigungswinkel von 30° ± 5°.

Besonders vorteilhaft ist eine Ausführungsform, bei der die Personenvermessungsstation zwei derartige Seitenwände aufweist, die den Messraum an zwei sich gegenüberliegenden Seiten begrenzen. Zweckmäßig sind diese beiden Seitenwände bezüglich der Mittelebene spiegelsymmetrisch angeordnet.

Sofern zwei derartige Seitenwände vorgesehen sind, lassen sich beide Seitenkonturen der jeweiligen Person vermessen. Hierdurch ist es zum einen möglich, besonders einfach die jeweils erforderliche Seitenkontur für solche Personen zu bestimmen, die eine bevorzugte Seitenlage während ihres Schlafs einnehmen. Zum anderen kann die Auswerteeinheit anhand beider Seitenkonturen den Verlauf der Wirbelsäule bezogen auf die Mittelebene berechnen. Hierdurch lassen sich evtl. Schieflagen oder Abweichungen der Wirbelsäule bezüglich der Mittelebene feststellen. Der Berater kann anhand einer solchen Schieflage bzw. Abweichung Empfehlungen für eine bevorzugte Seitenlage und/oder für ein geeignetes Kissen geben. Desweiteren lässt sich mit Hilfe der beiden Seitenkonturen eine Körperbreite ermitteln, die bspw. Rückschlüsse über das Gewicht der Person zulässt und die insbesondere eine Empfehlung für eine geeignete Matratze ermöglicht.

Die Auswerteeinheit kann gemäß einer vorteilhaften Ausführungsform ein Display aufweisen, über das bspw. an einer Darstellung eines Personenmodells die zu vermessenden Bereiche der Seite der zu vermessenden Person dargestellt sind. In dieser Darstellung können die bereits vermessenen Bereiche entsprechend markiert oder sogar bemaßt sein. Mit Hilfe des Displays lässt sich die Vermessung der jeweiligen Seitenkontur für eine Hilfsperson, die einerseits die Handhabung des Abstandsmessgeräts und andererseits die Bedienung der Personenvermessungsstation durchführt, erheblich vereinfachen. Insbesondere lassen sich dadurch auch Anweisungen generieren, die es sogar einem ungeübten Berater ermöglichen, eine präzise Vermessung der Seitenkontur der jeweiligen Person durchzuführen.

Besonders zweckmäßig ist eine Ausführungsform, bei welcher das Display in eine solche Seitenwand an einer dem Messraum zugewandten Seite integriert ist. Insbesondere kann das Display in die zuvor genannte Konvergenzzone der jeweiligen Seitenwand integriert sein. Hierdurch ergibt sich eine besonders gute Einsehbarkeit für die Hilfsperson auf das Display.

Bei einer vorteilhaften Ausführungsform kann die Standfläche auf einem horizontalen Boden der Personenvermessungsstation ausgebildet sein, der fest mit der Rückwand und der jeweiligen Seitenwand verbunden ist. Bei dieser Ausführungsform liegt eine hinsichtlich ihrer Struktur definierte Standfläche vor. Bei dieser Bauform ist die Personenvermessungsstation separat zu einem Zimmer oder Raum eines Gebäudes ausgestaltet, in dem sie angeordnet ist. Alternativ dazu ist auch eine Ausführungsform denkbar, bei welcher die Standfläche auf einem horizontalen Boden ausgebildet ist, auf dem die Personenvermessungsstation steht. Dieser Boden gehört dann zu dem Gebäude, in dessen Raum oder Zimmer die Personenvermessungsstation separat angeordnet ist oder integral ausgebildet ist.

Bei einer anderen Ausführungsform kann die Personenvermessungsstation außerdem eine horizontale Decke aufweisen, die fest mit der Rückwand und der jeweiligen Seitenwand verbunden ist. Mit Hilfe der Decke besitzt die Personenvermessungsstation eine erhöhte Stabilität, wodurch insbesondere auch die räumliche Lage der Rückwand zur jeweiligen Seitenwand definiert ist. Die Decke kann jedoch auch zur Größenmessung der jeweiligen Person herangezogen werden. Beispielsweise kann hierzu mit Hilfe des Abstandsmessgeräts der Abstand vom Kopf der Person, die auf der Standfläche Platz genommen hat, zur Decke gemessen werden. Die Auswerteeinheit kennt den Abstand von der Decke zur Standfläche und kann dann aus dem Abstand zwischen Kopf und Decke die Körpergröße berechnen. Die Körpergröße ist dann von Bedeutung, wenn mit Hilfe der ermittelten Seitenkontur Einstellparameter für die Unterfederung bereitgestellt werden sollen. Ferner kann beispielsweise auch die vertikale Position einer Schulter der Person über eine Abstandsmessung zur Decke ermittelt werden. Grundsätzlich kann zur Ermittlung einer vertikalen Position oder Personengröße auch eine Decke des Zimmers oder Raums herangezogen werden, in dem sich die Personenvermessungsstation befindet. Bedingung ist, dass der sich dabei ergebende Abstand in der Reichweite des Abstandsmessgeräts liegt. Über eine Initialmessung kann mit Hilfe des Abstandsmessgeräts der Abstand von der Standfläche zur Decke gemessen werden, so dass diese der Auswerteeinrichtung bekannt ist.

Bei einer anderen vorteilhaften Ausführungsform kann in die Rückwand eine Rückenkonturerfassungseinrichtung integriert sein, mit deren Hilfe eine Rückenkontur einer mit dem Rücken zur Rückwand auf der Standfläche stehenden Person erfasst werden kann. Während die Seitenkontur der Person für die Einstellung eines Liegesystems für Personen erforderlich ist, die bevorzugt auf der Seite schlafen, ist die Rückenkontur bei Personen, die bevorzugt auf dem Rücken schlafen, für die Einstellung des Liegesystems wichtig. Mit anderen Worten, mit Hilfe der Rückenkonturfassungseinrichtung wird die hier vorgestellte Personenvermessungsstation dahingehend erweitert, dass damit auch eine Rückenkontur erfasst werden kann. Durch die Integration der Rückenkonturerfassungseinrichtung in die Rückwand der Personenvermessungsstation ergibt sich eine raumsparende Unterbringung der Rückenkonturerfassungseinrichtung innerhalb der Personenvermessungsstation. Außerdem lassen sich, wenn die zu vermessende Person mit dem Rücken zur Rückwand auf der Standfläche steht, die Rückenkontur und die Seitenkontur quasi in einem einzigen Vermessungsvorgang innerhalb der Personenvermessungsstation durchführen. Die Rückenkonturerfassungseinrichtung kann vorzugsweise eine vormontierbare Baugruppe sein, die vorzugsweise als Ganzes nach Art eines Moduls optional in die Rückwand eingesetzt werden kann.

Gemäß einer vorteilhaften Weiterbildung kann die Rückenkonturerfassungseinrichtung eine Vielzahl von horizontal verstellbaren Schiebern aufweisen, die vertikal übereinander angeordnet sind und die einzeln von einer Ausgangsstellung, in der sie maximal in die Rückwand eingeschoben sind, in eine Messstellung verstellbar sind, in der sie bis zum Kontakt mit dem Rücken der Person aus der Rückwand in den Messraum hinein ausschiebbar sind. Mit Hilfe der übereinander angeordneten Schieber lässt sich somit einfach die Kontur des Rückens der auf der Standfläche stehenden Person nachbilden. Auch hierbei kann die Person bekleidet sein, da sich mit Hilfe der Schieber die Bekleidung leicht an den Körper andrücken lässt. Der Begriff "Vielzahl" bedeutet im vorliegenden Zusammenhang "mindestens zehn". Mit anderen Worten, die Rückenkonturerfassungseinrichtung weist zehn oder mehr Schieber auf.

Entsprechend einer Weiterbildung kann die Rückenkonturerfassungseinrichtung einen vorzugsweise vertikalen Messbalken aufweist, der eine dem Messraum zugewandte Vorderseite aufweist, die insbesondere vertikal und eben ist sowie gegenüber einer dem Messraum zugewandten Oberfläche der Rückwand nach hinten versetzt angeordnet ist, so dass mit dem Abstandsmessgerät eine Ausfahrlänge wenigstens eines solchen in die Messstellung verstellten Schiebers gegenüber der Vorderseite des Messbalkens messbar ist. Da das Abstandsmessgerät selbst eine gewisse Länge besitzt, muss die Ausfahrlänge größer sein als die Länge des Messgeräts, damit sie überhaupt mit dem Abstandsmessgerät messbar ist. Durch die gegenüber der Oberfläche der Rückwand vertiefte Anordnung des Messbalkens wird die Ausfahrlänge gegenüber der Oberfläche der Rückwand vergrößert, was die Messung mit dem Abstandsmessgerät vereinfacht bwz. ermöglicht.

Besonders vorteilhaft ist eine Weiterbildung, bei welcher der jeweilige Schieber im Bereich einer vertikalen Mittelebene der Personenvermessungsstation eine Aussparung aufweist, die so bemessen ist, dass das Abstandsmessgerät in diese Aussparung einführbar ist, wobei der Messbalken innerhalb der Aussparungen angeordnet ist. In der Folge kann die Messung der Ausfahrlänge bei in die Aussparung wenigstens eines Schiebers eingesetztem Abstandsmessgerät durch diese Aussparungen zum Messbalken horizontal durchgeführt werden.

Vorteilhaft ist auch eine Weiterbildung, bei welcher die Auswerteeinrichtung so ausgestaltet ist, dass sie zum Vermessen der Rückenkontur zumindest einen Rückenbereich vorgibt, in dem mittels des Abstandsmessgeräts der Abstand eines dem vorgegebenen Rückenbereich zugeordneten Schiebers zu einem die Standfläche aufweisenden horizontalen Boden, insbesondere des Gebäudes, oder zu einer horizontalen Decke, insbesondere der Personenvermessungsstation, gemessen werden soll. Beispielsweise kann als Rückenbereich, dessen Abstand zum Boden bzw. zur Decke ermittelt werden soll, durch den Lordosenbereich des Rückens gebildet sein. Der Lordosenbereich enthält die Lordose der Wirbelsäule. Die Lordose schließt an das Becken an und ist durch die nach ventral, also zum Bauch hin konvexe Krümmung der Wirbelsäule gebildet. Durch die Vermessung des vorgegebenen Rückenbereichs kennt die Auswerteeinheit die Position dieses Rückenbereichs innerhalb der Größe, also in der Höhenrichtung der Person und kann die Seitenkontur entsprechend dem Höhenverlauf der Person zuordnen.

Zweckmäßig kann der jeweilige Schieber plattenförmig ausgestaltet sein und jeweils in einer horizontalen Ebene liegen. Ferner kann der jeweilige Schieber im Bereich einer vertikalen Mittelebene der Personenvermessungsstation eine Aussparung aufweisen, die so dimensioniert ist, dass das Abstandsmessgerät in diese Aussparung einführbar ist, so dass insbesondere die Abstandsmessung zum Boden oder zur Decke durch diese Aussparungen der Schieber hindurch durchführbar ist. Auch diese Maßnahme vereinfacht die Handhabung des Abstandsmessgeräts in Verbindung mit der Rückenkonturerfassungseinrichtung. Der jeweilige Schieber kann dann in einer vertikalen Draufsicht U-förmig ausgestaltet sein.

Gemäß einer anderen vorteilhaften Ausführungsform, bei der die Personenvermessungsstation zwei solche Seitenwände aufweist, die den Messraum an zwei sich gegenüberliegenden Seiten begrenzen, kann die Auswerteeinheit so ausgestaltet bzw. programmiert sein, dass sie die Bereiche, deren Abstand zur jeweiligen Seitenwand zu messen ist, für beide Seiten der Person vorgibt. Anhand der mit Hilfe des Abstandsmessgeräts gemessenen und an die Auswerteeinheit übermittelten Abstände kann die Auswerteeinheit dann die beiden Seitenkonturen der Person ermitteln. Ferner kann die Auswerteeinheit so ausgestaltet bzw. programmiert sein, dass sie anhand der beiden Seitenkonturen einen Breitenverlauf der Person und/oder einen Verlauf der Wirbelsäule der Person entlang der Mittelebene ermitteln kann.

Die Erfindung betrifft außerdem eine Betteinstelleinrichtung zum Einstellen eines Betts für eine Person, die mit einer Personenvermessungsstation der vorstehend beschriebenen Art ausgestattet ist. Die Betteinstelleinrichtung ist außerdem mit einer Einstellungsüberprüfungsstation ausgestattet, die ein Bett mit einem Liegesystem, vorzugsweise mit einer Unterfederung und einer Matratze, aufweist. Das Liegesystem kann nun abhängig von der in der Personenvermessungsstation ermittelten Seitenkontur einstellbar sein, um das Liegesystem an die Seitenkontur der Person anzupassen. Die Anpassung des Liegesystems an die Seitenkontur kann dabei manuell oder bei einer komfortablen Ausführungsform mit Hilfe von Stellmotoren und insbesondere automatisch erfolgen. Beispielsweise kann dann die Auswerteeinheit diese Stellmotoren ansteuern oder ein Steuergerät der Einstellungsüberprüfungsstation ansteuern, das seinerseits die Stellmotoren ansteuert, um die jeweilige Einstellung durchzuführen.

Die Auswerteeinheit der Personenvermessungsstation kann nun weiter so ausgestaltet und/oder programmiert sein, dass sie zum Überprüfen der Einstellung des Liegesystems mehrere Bereiche der Person vorgibt, in denen jeweils der Abstand zu einer horizontalen Decke zu messen ist, wenn die Person in Seitenlage auf der Matratze liegt. Die jeweilige Abstandsmessung kann dabei wieder mit Hilfe des Abstandsmessgeräts der Personenvermessungsstation durchgeführt werden. Auch hier ist das Abstandsmessgerät dann zweckmäßig so ausgestaltet, dass es für den jeweiligen Bereich den gemessenen Abstand automatisch an die Auswerteeinheit übermittelt. Schließlich ist die Auswerteeinheit so ausgestaltet bzw. programmiert, dass sie anhand der den einzelnen Bereichen zugeordneten Abstände einen Ist-Verlauf der Wirbelsäule der Person und die Abweichung dieses Ist-Verlaufs von einem Soll-Verlauf ermittelt. Der Berater, der für die vermessene Person die Unterfederung einstellt, kann nun anhand der Abweichungen überprüfen, ob die Einstellung der Unterfederung in Ordnung ist und ob sowie ggf. wo noch eine Anpassung oder Korrektur des Liegesystems durchgeführt werden muss.

Besonders vorteilhaft ist eine Weiterbildung, bei welcher die Auswerteeinheit so ausgestaltet bzw. programmiert ist, dass sie anhand der ermittelten Seitenkontur Einstellparameter für das Liegesystem ermittelt, die dazu führen, dass sich für die Wirbelsäule der Person, wenn sie in Seitenlage auf einem Referenzbett liegt, dessen Unterfederung mit diesen Einstellparameters eingestellt ist, der Soll-Verlauf einstellen soll. Mit anderen Worten, die Auswerteeinheit gibt nicht oder nicht nur die Seitenkontur der vermessenen Person aus, sondern generiert die Einstellparameter, die erfahrungsgemäß zu einer optimalen Anpassung des Liegesystems führen sollen. Hierdurch vereinfacht sich für den Berater die Anpassung des Betts bzw. des Liegesystems, vorzugsweise der Unterfederung, an die jeweilige Person. Die Zuordnung der Einstellparameter zu der gemessenen Seitenkontur kann mit einem Algorithmus oder mit einem Kennfeld erfolgen, in dem Erfahrungswerte hinterlegt sind.

Besonders vorteilhaft ist nun eine Weiterbildung, bei welcher die Auswerteeinheit so ausgestaltet bzw. programmiert ist, dass sie abhängig vom ermittelten Ist-Verlauf automatisch Korrekturparameter für das Liegesystem ermittelt, die zu einer Reduzierung und im Idealfall zu einer Eliminierung der Soll-Ist-Differenz führen. Auch diese Maßnahme erleichtert es dem Berater, die optimale Einstellung für das Liegesystem zu finden. Abweichungen zwischen Ist-Verlauf und Soll-Verlauf können vor allem durch das tatsächliche Gewicht der jeweiligen Person und/oder durch die Elastizität bzw. Härte der jeweils verwendeten Matratze entstehen. Während bei der Vermessung der Seitenkontur Erfahrungswerte berücksichtigt werden, die zu Einstellparametern führen, bei denen erfahrungsgemäß der gewünschte Soll-Verlauf erreicht werden kann, wirkt sich das tatsächliche Gewicht der Person bzw. die Matratzenhärte in der Seitenlage signifikant auf den tatsächlichen Verlauf, also den Ist-Verlauf der Wirbelsäule aus.

Die Erfindung beruht beim Verfahren auf dem allgemeinen Gedanken, die jeweilige Seitenkontur einer Person mit Hilfe eines Abstandsmessgeräts zu vermessen, indem das Abstandsmessgerät mit der Seite der jeweiligen Person in Kontakt gebracht wird und der Abstand von dieser Seite der Person zu einer vorbestimmten Seitenwand mit Hilfe des Abstandsmessgeräts berührungslos gemessen wird. Auf diese Weise wird quasi von der Person weg gemessen auf eine Referenzebene hin, wodurch quasi ein Negativabdruck der Seitenkontur gemessen und durch entsprechende Umrechnung die tatsächliche Seitenkontur ermittelt wird. Die Vermessung von der Person weg hat den Vorteil, dass sämtliche Fehler, die durch die Bekleidung der Person entstehen können, weitgehend ausgeschlossen werden können. Somit ist es insbesondere möglich, die Person im bekleideten Zustand zu vermessen. Zum Einen lassen sich dadurch die Messungen leichter durchführen. Zum Anderen erleichtert es den Personen, Hemmungen zu überwinden, die der Durchführung der Vermessung entgegenstehen können.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile beziehen.

Es zeigen, jeweils schematisch,
- Fig. 1: eine isometrische Ansicht einer Personenvermessungsstation,
- Fig. 2: eine Frontansicht der Personenvermessungsstation,
- Fig. 3: eine Seitenansicht der Personenvermessungsstation entsprechend einer Blickrichtung III in Fig. 2,
- Fig. 4: einen Längsschnitt der Personenvermessungsstation entsprechend Schnittlinien IV in Fig. 2,
- Fig. 5: einen Querschnitt der Personenvermessungsstation entsprechend Schnittlinien V in Fig. 2,
- Fig. 6: eine stark vereinfachte Ansicht der Personenvermessungsstation im Bereich eines Abstandsmessgeräts beim Messen eines Abstands,
- Fig. 7: eine stark vereinfachte Seitenansicht der Personenvermessungsstation im Bereich einer Rückenkonturerfassungseinrichtung beim Vermessen einer Rückenkontur,
- Fig. 8: eine stark vereinfachte Ansicht einer Betteinstelleinrichtung.

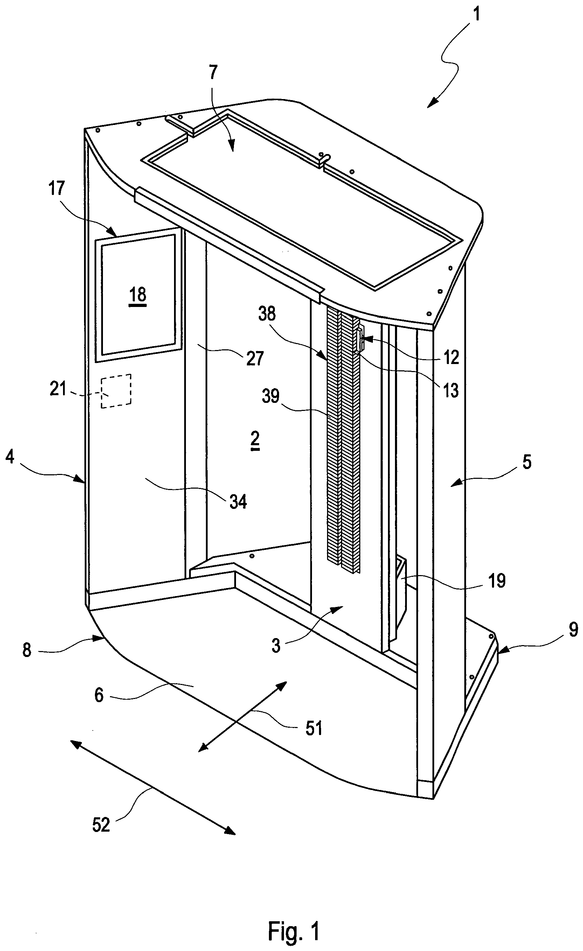

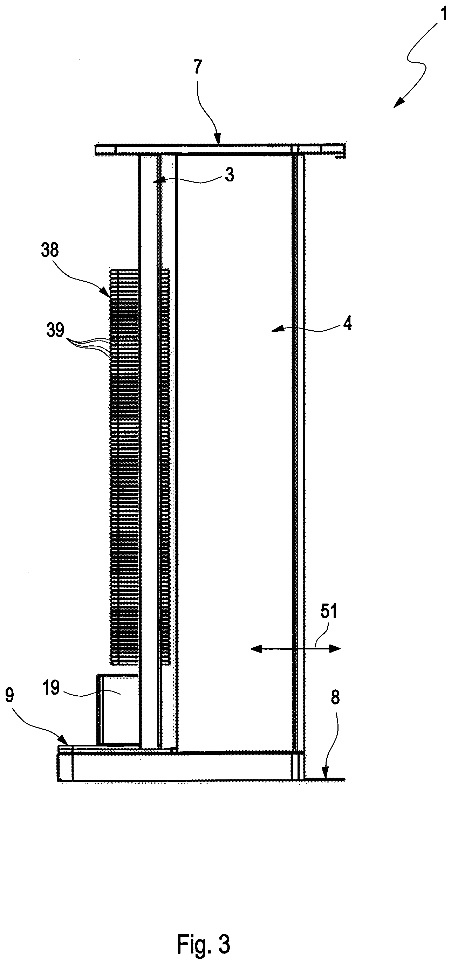

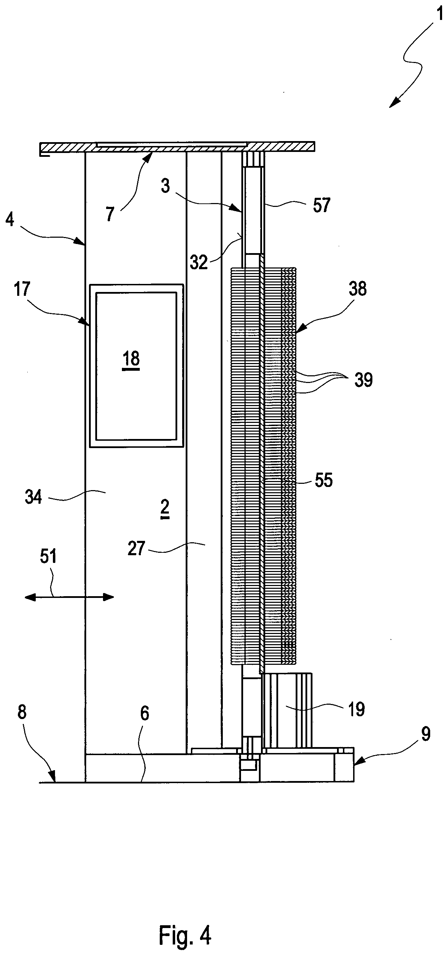

Entsprechend den Fig. 1 bis 5 umfasst eine Personenvermessungsstation 1 einen Messraum 2, eine vertikale Rückwand 3, zumindest eine vertikale Seitenwand 4, 5 und eine horizontale Standfläche 6. Im gezeigten, bevorzugten Beispiel besitzt die Personenvermessungsstation 1 zwei Seitenwände 4, 5, die sich am Messraum 2 gegenüberliegen. Außerdem ist eine Decke 7 vorgesehen, die fest mit den beiden Seitenwänden 4, 5 und mit der Rückwand 3 verbunden ist. Im Beispiel weist die Personenvermessungsstation 1 außerdem einen Boden 8 auf, der fest mit den beiden Seitenwänden 4, 5 und mit der Rückenwand 3 verbunden ist. Der Boden 8 weist die Standfläche 6 auf. Ferner ist im gezeigten Beispiel eine Konsole 9 vorgesehen, die fest mit dem Boden 8, mit der Rückwand 3 und mit beiden Seitenwänden 4, 5 verbunden ist und die für eine erhöhte Standfestigkeit der Personenvermessungsstation 1 sorgt. Die Rückwand 3 begrenzt den Messraum 2 nach hinten. Die beiden Seitenwände 4, 5 begrenzen den Messraum 2 jeweils seitlich. Die Standfläche 6 bzw. der Boden 8 begrenzt den Messraum 2 nach unten. Die Decke 7 begrenzt den Messraum 2 nach oben. Sofern die Personenvermessungsstation 1 keinen eigenen Boden 8 besitzt, befindet sich die Standfläche 6 auf einem Boden, auf dem die Personenvermessungsstation 1 insgesamt steht. Die relativen Begriffe "oben", "unten", "seitlich", "vorne" und "hinten" beziehen sich im vorliegenden Zusammenhang auf den betriebsbereiten Zustand der Personenvermessungsstation 1. Eine in den Fig. 1 und 3 bis 5 durch einen Doppelpfeil angedeutete Tiefenrichtung 51 erstreckt sich somit horizontal und von vorn nach hinten oder umgekehrt. Eine in den Fig. 1, 2 und 5 durch einen Doppelpfeil angedeutete Breitenrichtung 52 erstreckt sich somit horizontal und quer zur Tiefenrichtung 51, also seitlich bzw. von links nach rechts oder umgekehrt.

Im hier gezeigten bevorzugten Beispiel ist die Personenvermessungsstation 1 eine separate Einheit, die in einem Zimmer oder Raum eines Gebäudes angeordnet ist, um darin einen definierten Messraum 2 bereit zu stellen. Die genannten Wände 3, 4, 5 der Personenvermessungsstation 1 befinden sich dann innerhalb des Zimmers oder Raums, sind also zusätzlich zu Gebäudewänden vorgesehen, die das Zimmer bzw. den Raum des Gebäudes begrenzen.

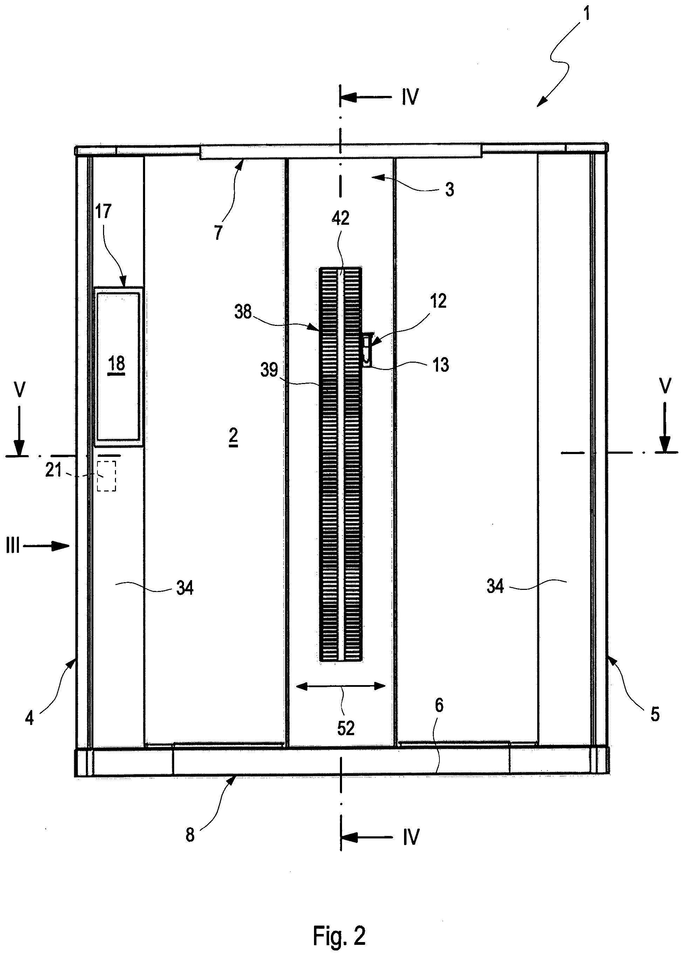

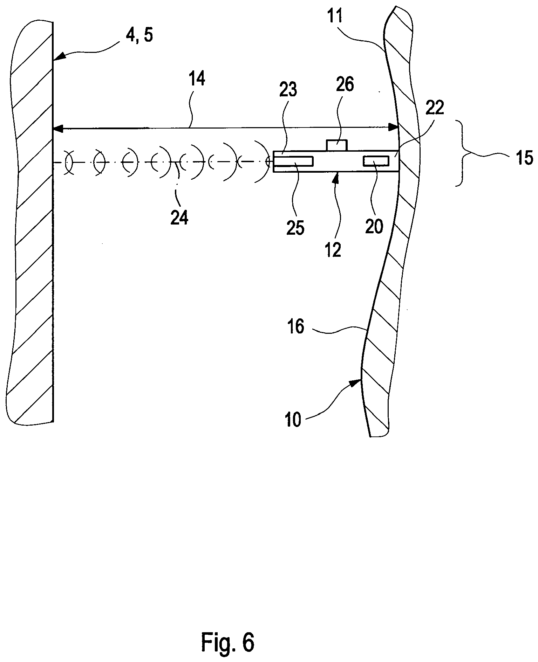

Auf der Standfläche 6 kann eine in Fig. 6 angedeutete Person 10 Platz nehmen, um eine Seitenkontur 11 der Person 10 zu vermessen. Hierzu positioniert sich die Person 10 auf der Standfläche 6 in einer Messposition, in der ein Rücken der Person 10 der Rückwand 3 zugewandt ist. Grundsätzlich ist auch denkbar, dass die Person 10 in der Messposition den Rücken von der Rückwand 3 abgewandt hat. Bevorzugt ist jedoch eine Messposition, bei welcher der Rücken der Person 10 der Rückwand 3 zugewandt ist. Jedenfalls ist eine Seite 16 der Person 10 der jeweiligen Seitenwand 4, 5 zugewandt. Wenn sich die Person 10 mit dem Rücken zur Rückwand 3 auf die Standfläche 6 stellt, ist eine rechte Seite 16 der Person 10 der in den Fig. 1 und 2 links wiedergegebenen Seitenwand 4 zugewandt, während eine linke Seite 16 der Person 10 der in den Fig. 1 und 2 rechts wiedergegebenen Seitenwand 5 zugewandt ist.

Die Personenvermessungsstation 1 weist außerdem zumindest ein Abstandsmessgerät 12 auf, das in den Fig. 1 und 2 in einer Halterung 13 angeordnet ist, die sich z.B. an der Rückwand 3 befindet. In dieser Halterung 13 kann das Abstandsmessgerät 12 abgelegt werden, wenn es nicht benötigt wird. Für die Durchführung einer Abstandsmessung kann eine Hilfsperson, welche die Messungen durchführt, das Abstandsmessgerät 12 in die Hand nehmen. Es handelt sich somit um ein manuell betätigbares und manuell tragbares Abstandsmessgerät 12, ein so genanntes "Handheld". Gemäß Fig. 6 kann mit Hilfe des Abstandsmessgeräts 12 ein Abstand 14 von einem vorbestimmten Bereich 15 der jeweiligen Seite 16 der Person 10 zu der dieser Seite 16 zugewandten Seitenwand 4 bzw. 5 gemessen werden.

Die Personenvermessungsstation 1 weist außerdem eine Auswerteeinheit 17 auf, die mit dem Abstandsmessgerät 12 zur Signalübertragung gekoppelt ist. Zweckmäßig kommunizieren Abstandsmessgerät 12 und Auswerteinheit 17 drahtlos. Die Auswerteeinheit 17 besitzt ein Display 18, an dem bspw. eine Anleitung zur Durchführung der jeweiligen Vermessung angezeigt werden kann. Ferner lassen sich am Display 18 die ermittelten Messdaten anzeigen. Das Display 18 ist in eine der beiden Seitenwände 4, 5 integriert und dem Messraum 2 zugewandt. Die Auswerteeinheit 17 kann eine hier nicht gezeigte Recheneinheit sowie weitere elektronische Komponenten wie eine Leistungselektronik und dergleichen aufweisen, die ebenfalls im Bereich einer der Seitenwände 4, 5 angeordnet sein können. Ebenso ist eine Unterbringung dieser Komponenten der Auswerteeinheit 17 in der Konsole 9 denkbar. Im Beispiel ist an einer vom Messraum 2 abgewandten Rückseite der Rückwand 3 eine Box 19 ausgebildet, die sich ebenfalls zur Unterbringung einer oder mehrerer oder sämtlicher Komponenten der Auswerteeinheit 17 eignet, abgesehen vom Display 18.

Die Auswerteeinheit 17 kann zum Vermessen der seitlichen Kontur 11 oder Seitenkontur 11 der Person 10 mehrere Bereiche 15 der Seite 16 der Person 10 vorgegeben, in denen jeweils der Abstand 14 zur Seitenwand 4 bzw. 5 gemessen werden soll. Die einzelnen Bereiche 15 sind in der Vertikalen, also in der Höhenrichtung voneinander beabstandet. Beispielsweise kann jeweils ein derartiger Bereich 15 auf Höhe des Kopfes, auf Höhe der Schultern, auf Höhe der Brust, auf Höhe der Taille, auf Höhe des Beckens und auf Höhe der Knie vorgesehen sein. Im Display 18 kann die Auswerteeinheit 17 den aktuell zu vermessenden Bereich 15 visualisieren, um der Hilfsperson die Durchführung der Vermessung zu erleichtern.

Gemäß Fig. 6 kann die Hilfsperson das Abstandsmessgerät 12 im jeweils zu vermessenden Bereich 15 an die Seite 16 der Person 10 anlegen und von dort aus den Abstand 14 zur jeweiligen Seitenwand 4, 5 messen. Das Abstandsmessgerät 12 übermittelt dann den gemessenen Abstand 14 automatisch an die Auswerteeinheit 17. Ein entsprechender Sender ist in Fig. 6 mit 20 bezeichnet, ein geeigneter Empfänger ist in den Fig. 1 und 2 mit unterbrochener Linie angedeutet und mit 21 bezeichnet. Wie erwähnt, kommunizieren Sender 20 und Empfänger 21 vorzugsweise drahtlos. Sender 20 und Empfänger 21 können jeweils kombinierte Sende- und Empfängereinheiten 20, 21 sein, bspw. kann dadurch die Auswerteeinheit 17 den empfangenen Messwert an das Abstandsmessgerät 12 zurücksenden, um diesen überprüfen und bestätigen zu lassen.

Die Auswerteeinheit 17 kann nun anhand der Abstände 14, die den einzelnen Bereichen 15 zugeordnet sind, die Seitenkontur 11 der jeweiligen Person 10 ermitteln. Es ist klar, dass die Auswerteeinheit 17 dabei Plausibilitätsprüfungen durchführen kann, beispielsweise um zu erkennen, wenn die Hilfsperson einen zu vermessenden Bereich übersprungen oder doppelt gemessen hat. Um die einzelnen seitlichen oder horizontalen Abstandswerte der horizontalen Messpunkte auch vertikalen Positionen zuordnen zu können, kann eine Abschätzung anhand der Größe der jeweiligen Person vorgenommen werden. Hierzu wird die Größe der Person der Auswerteeinheit 17 mitgeteilt. Vorzugsweise kann die Körpergröße mittels des Abstandsmessgeräts 12 gemessen und automatisch der Auswerteeinheit 17 übermittelt werden. Darüber hinaus ist es ebenso möglich, die vertikale Position des jeweiligen horizontalen Messpunkts separat zu ermitteln, vorzugsweise mit dem Abstandsmessgerät 12. Hierzu kann das Abstandsmessgerät 12 am jeweiligen Messpunkt auch vertikal ausgerichtet werden, um den vertikalen Abstand dieses Messpunkts von der Standfläche oder von der Decke 7 zu messen.

Gemäß Fig. 6 besitzt das Abstandsmessgerät 12 ein hinteres Ende 22 und ein vorderes Ende 23, die in einer Längsrichtung des Abstandsmessgeräts 12 voneinander entfernt sind. Für die jeweilige Abstandsmessung wird das Abstandsmessgerät 12 mit seinem hinteren Ende 22 an der Seite 16 der Person 10 in Kontakt gebracht. Dabei kann die Hilfsperson behutsam Kleidung der Person 10 gegen die Haut der Person 10 andrücken, so dass die Seitenkontur 11 weitgehend unbeeinträchtigt von der vergleichsweise dünnen Kleidung gemessen werden kann. Am vorderen Ende 23 erfolgt dann eine berührungslose Messung des Abstands 14 zur jeweiligen Seitenwand 4, 5. Zu dieser horizontalen Abstandsmessung wird das Abstandsmessgerät 12 horizontal ausgerichtet. Zweckmäßig kann das Abstandsmessgerät 12 hierzu an seinem vorderen Ende 23 einen Messstrahl 24 ausstrahlen, der auf die jeweilige Seitenwand 4, 5 auftrifft. Außerdem kann das Abstandsmessgerät 12 eine Messeinheit 25 enthalten, die eine zum Abstandsmessgerät 12 zurückgestrahlte Reflexion des Messstrahls 24 detektiert und dabei die Zeit misst, die der Messstrahl 24 vom Abstandsmessgerät 12 zur Seitenwand 4, 5 und zurück benötigt. Anhand dieser Zeit kann die Messeinheit 25 dann den Abstand 14 vom hinteren Ende 22 zur Seitenwand 4, 5 berechnen. Beispielsweise arbeitet das Abstandsmessgerät 12 hierzu mit einem Ultraschallstrahl oder mit einem Laserstrahl. Mit einem Betätigungselement 26, z. Bsp. in Form eines Tasters, kann die Hilfsperson die Abstandsmessung auslösen. Ein optisches und/oder akustisches Signal kann die erfolgreiche Übermittlung einer Abstandsmessung zur Auswerteeinheit 17 signalisieren. Der Messstrahl 24 kann bei einer Lasermessung sichtbar sein. Falls der Messstrahl 24 selbst für das menschliche Auge unsichtbar ist, kann das Abstandsmessgerät 12 auch so konzipiert sein, dass es einen sichtbaren Hilfsstrahl nach Art eines Laserpointers abstrahlt. Dieser Hilfsstrahl verläuft zweckmäßig koaxial zum Messstrahl 24.

Gemäß den Fig. 1 bis 5 besitzt die jeweilige Seitenwand 4, 5 jeweils eine Messzone 27, die sich an einem der Rückwand 3 zugewandten hinteren Ende 28 der jeweiligen Seitenwand 4, 5 befindet. Die jeweilige Messzone 27 weist eine dem Messraum 2 zugewandte Oberfläche 29 auf, die jeweils in einer vertikalen Seitenebene 30 liegt. Die jeweilige Seitenebene 30 erstreckt sich ihrerseits jeweils senkrecht zu einer Rückseitenebene 31, in der eine dem Messraum 2 zugewandte Oberfläche 32 der Rückwand 3 liegt. Insbesondere verlaufen die beiden Seitenebenen 30 somit parallel zueinander und parallel zu einer Mittelebene 33, die sich quer zur Tiefenrichtung 51 mittig durch die Rückwand 3 und mittig durch die Personenvermessungsstation 1 erstreckt. Außerdem besitzt die jeweilige Seitenwand 4, 5 hier eine Konvergenzzone 34, die innerhalb der jeweiligen Seitenwand 4, 5 nach vorn an die zugehörige Messzone 27 angrenzt. Die jeweilige Konvergenzzone 34 besitzt eine dem Messraum 2 zugewandte Oberfläche 35, die jeweils in einer vertikalen Konvergenzebene 36 liegt. Die beiden Konvergenzebenen 36, der beiden Konvergenzzonen 34 sind jeweils gegenüber der ebenfalls vertikalen Mittelebene 33 so geneigt, dass sie sich hinter der Rückwand 3, also an einer vom Messraum 2 abgewandten Seite der Rückwand 3 in einer vertikalen Schnittgeraden 37 schneiden. Ein Neigungswinkel α zwischen der jeweiligen Konvergenzebene 36 und der Mittelebene 33, der auch an der jeweiligen Seitenwand 4, 5 zwischen der zugehörigen Konvergenzebene 36 und der zugeordneten Seitenebene 30 vorliegt, besitzt im Beispiel einen Wert von etwa 30°. Es ist klar, dass grundsätzlich auch andere Werte für den Neigungswinkel α vorliegen können. Bevorzugt liegt der Neigungswinkel α in einem Wertebereich von etwa 20° bis etwa 70°.

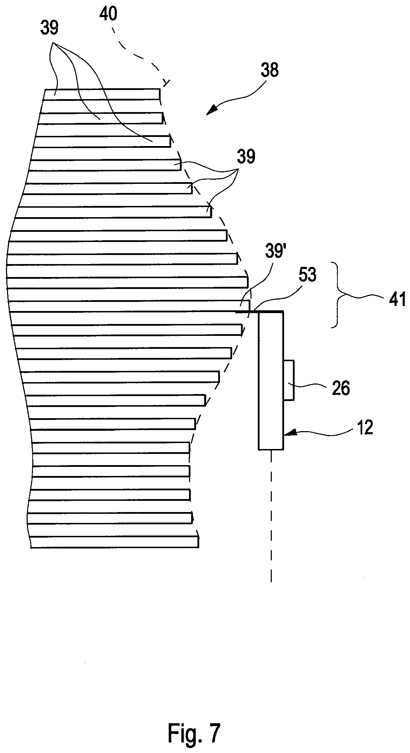

Die hier vorgestellte Personenvermessungsstation 1 ist außerdem mit einer Rückenkonturerfassungseinrichtung 38 ausgestattet, die zum Erfassen einer Rückenkontur einer mit dem Rücken zur Rückwand 3 auf der Standfläche 6 stehenden Person 10 dient und die in die Rückwand 3 integriert ist. Die Rückenkonturerfassungseinrichtung 38 weist im gezeigten Beispiel eine Vielzahl von horizontal verstellbaren Schiebern 39 auf, die vertikal übereinander angeordnet sind und die einzeln von einer Ausgangsstellung, die in den Fig. 1 bis 5 wiedergegeben ist, in eine Messstellung verstellbar sind, die exemplarisch für einige der Schieber 39 in Fig. 7 widergegeben ist. In der Ausgangsstellung sind die Schieber 39 maximal in die Rückwand 3 eingeschoben und dadurch weitgehend aus dem Messraum 2 heraus verstellt. Für die jeweilige Messstellung, in der sie bis zum Kontakt mit dem Rücken der Person 10 aus der Rückwand 3 ausschiebbar sind, werden die Schieber 39 mehr oder weniger weit in den Messraum 2 hinein verstellt.

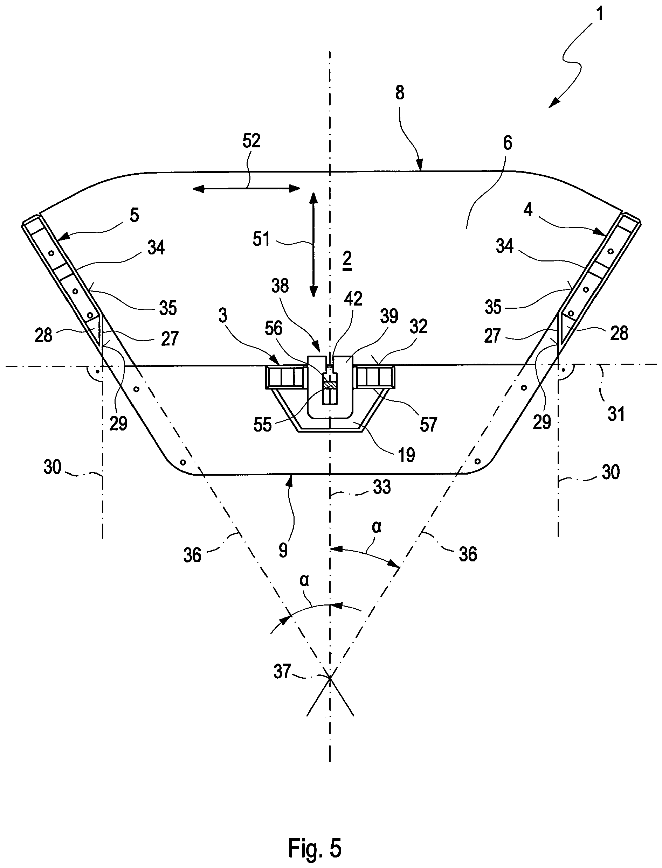

Rein exemplarisch ist in Fig. 7 ein Ausschnitt der Rückenkonturerfassungseinrichtung 38 widergegeben, bei der alle erkennbaren Schieber 39 in die Messstellung überführt sind, um eine Rückenkontur 40 nachzubilden, die in Fig. 7 durch eine unterbrochene Linie angedeutet ist. Die Auswerteeinheit 17 kann nun für die Vermessung der Rückenkontur 40 zumindest einen Rückenbereich 41 vorgeben, in dem mittels des Abstandsmessgeräts 12 der Abstand eines Schiebers 39', der dem vorgegebenen Rückenbereich 41 zugeordnet ist, zum Boden 6 oder alternativ zur Decke 7 gemessen werden soll. Damit diese vertikale Abstandsmessung möglichst einfach mit hoher Genauigkeit durchgeführt werden kann, kann gemäß den Fig. 2 und 5 vorgesehen sein, dass die Schieber 39 jeweils plattenförmig ausgestaltet sind und dabei in einer horizontalen Ebene liegen. Im Beispiel ist am Abstandsmessgerät 12 außerdem eine Hilfskontur 53 am hinteren Ende 22 angebracht, z.B. in Form eines Winkels, um das Abstandsmessgerät 12 besser an dem jeweiligen Schieber 39 für die vertikale Abstandsmessung positionieren zu können.

Ferner weist hier der jeweilige Schieber 39 im Bereich der Mittelebene 33 eine Aussparung 42 auf, die so groß ist, dass das Abstandsmessgerät 12 in diese Aussparungen 42 einführbar ist. In der Folge kann die vertikale Abstandsmessung zum Boden 8 bzw. zur Decke 7 durch diese Aussparungen 42 des Schiebers 39 hindurch erfolgen. Dies hat den Vorteil, dass das Abstandsmessgerät 12 vergleichsweise exakt vertikal ausgerichtet werden muss, damit der Messstrahl 24 innerhalb der Aussparungen 42 bis zum Boden 8 bzw. bis zur Decke 7 gelangen kann. In der Folge besitzt der gemessene Abstand auch eine hohe Genauigkeit. Zur Realisierung der Aussparung 42 kann der jeweiligen Schieber 39 gemäß Fig. 5 in der vertikalen Draufsicht U-förmig ausgestaltet sein. Auch wenn das Abstandsmessgerät 12 mit Ultraschall zur Abstandsmessung arbeitet, kann es mit einer Laserdiode ausgestattet sein, um die Stelle optisch zu markieren, gegenüber der die aktuelle Abstandsmessung erfolgt.

Des Weiteren kann mit Hilfe des Abstandsmessgeräts 12 auch eine Ausfahrlänge bestimmter Schieber 39 oder aller Schieber 39 gemessen werden. Die Ausfahrlänge entspricht dabei dem Weg, den der jeweilige Schieber 39 zurücklegt, wenn er von seiner Ausgangsstellung in die Messstellung überführt wird. Um die Messung der Ausfahrlänge mit Hilfe des Abstandsmessgeräts besser durchführen zu können, kann die Rückenkonturerfassungseinrichtung 38 gemäß Fig. 5 mit einem Messbalken 55 ausgestattet sein, der ebenfalls in der Rückwand 3 angeordnet ist. Der Messbalken 55 weist eine dem Messraum 2 zugewandte Vorderseite 56 auf, die gegenüber der Oberfläche 32 der Rückwand 3 nach hinten versetzt angeordnet ist. Somit kann mit dem Abstandsmessgerät 12 die Ausfahrlänge wenigstens eines solchen in die Messstellung verstellten Schiebers 39 gegenüber der Vorderseite 56 des Messbalkens 55 gemessen werden. An dieser Stelle steht dem Abstandsmessgerät 12 mehr horizontaler Platz zur Verfügung als beispielsweise bei einer Messung gegenüber der Oberfläche 32 der Rückseite 3. Der Messbalken 55 ist hierbei innerhalb der Aussparungen 42 angeordnet und kann für diese zusätzlich eine Führungsfunktion besitzen. Insbesondere ist es dabei auch möglich, das Abstandsmessgerät 12 in zumindest eine solche Aussparung 42 einzuführen, um dort den horizontalen Abstand zur Vorderseite 56 des Messbalkens 55 zu messen. Aus dieser Abstandsmessung lässt sich dann ohne weiteres die Ausfahrlänge bestimmen. Im gezeigten Beispiel ist der Messbalken 55 an seiner Rückseite bündig zu einer Rückseite 57 der Rückwand 3 angeordnet.

Sofern die Personenvermessungsstation 1 wie hier mit zwei gegenüberliegenden Seitenwänden 4, 5 ausgestattet ist, kann die Auswerteeinheit 17 so konfiguriert sein, dass sie die Bereiche 15, in denen der Abstand 14 zur jeweiligen Seitenwand 4, 5 gemessen werden soll, für beide Seiten 16 der Person 10 vorgibt. In der Folge werden kann in Verbindung mit dem Abstandsmessgerät 12 beide Seitenkonturen 11 der Person 10 ermittelt, also deren linke und rechte Seitenkontur 16. Die Auswerteeinheit 17 kann aus beiden Seitenkonturen bspw. einen Breitenverlauf, vorzugsweise jedoch einen Verlauf der Wirbelsäule der Person 10 ermitteln. Weicht die Wirbelsäule übermäßig von der Mittelebene 33 ab, bspw. durch eine Neigung oder Krümmung, kann die Auswerteeinheit 17 über das Display 18 ein entsprechendes Signal geben. Der Berater kann die Person 10 entsprechend aufklären und geeignete Empfehlungen aussprechen.

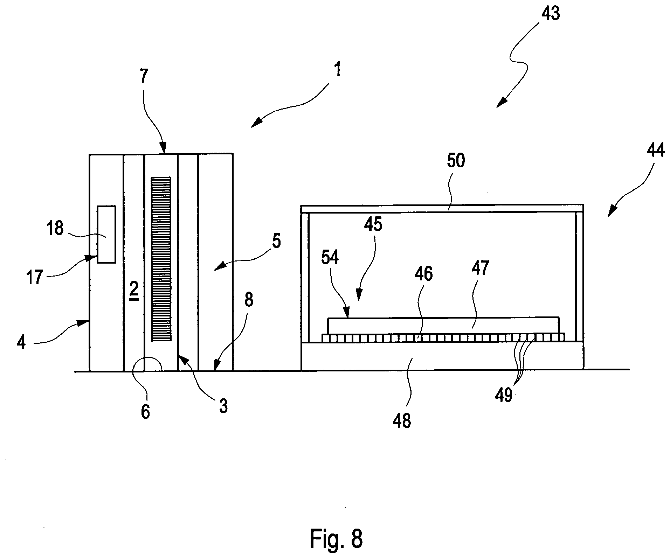

Entsprechend Fig. 8 kann eine Betteinstelleinrichtung 43 eine Personenvermessungsstation 1 der vorstehend beschriebenen Art sowie eine Einstellungsüberprüfungsstation 44 aufweisen. Die Einstellüberprüfungsstation 44 umfasst ein Bett 45, das mit einem Liegesystem 54 ausgestattet ist, das seinerseits zumindest eine Unterfederung 46 und eine Matratze 47 aufweist. Im Beispiel der Fig. 8 weist das Bett 45 außerdem ein Gestell 48 auf, das das Liegesystem 54 trägt. Im Beispiel trägt somit das Gestell 48 die Unterfederung 46, während die Unterfederung 46 ihrerseits die Matratze 47 trägt. Die Unterfederung 46 kann bspw. durch einen Lattenrost gebildet sein, der sich durch eine Vielzahl einzelner Latten 49 auszeichnet. Das Liegesystem 54, also insbesondere Unterfederung 46 und Matratze 47 lassen sich individuell auf die jeweilige Person 10 einstellen, um einen möglichst hohen Schlafkomfort zu erzielen. Die Matratze 47 lässt sich hinsichtlich Härte und Dicke wählen, außerdem kommen unterschiedlichste Materialien in Betracht. Ferner sind auch Matratzen 47 bekannt, die in verschiedenen Liegezonen unterschiedliche Dicken und Härten besitzen. Die Unterfederung 46 kann bspw. hinsichtlich ihrer Federelastizität über die gesamte Länge des Betts 45 eingestellt werden. Hierzu können die einzelnen Latten 49 hinsichtlich ihrer Federsteifigkeit variiert werden. Somit kann das Liegesystem 54, insbesondere die Unterfederung 46, abhängig von der in der Personenvermessungsstation 1 ermittelten Seitenkontur 11 eingestellt werden, um das Liegesystem 54, insbesondere die Unterfederung 46, an diese Seitenkontur 11 der Person 10 anzupassen.

Die Auswerteeinheit 17 der Personenvermessungsstation 1 kann nun ferner so ausgestaltet bzw. programmiert sein, dass sie zum Überprüfen der Einstellung des Liegesystems 54 bzw. der Unterfederung 46 mehrere Bereiche der Person 10 vorgibt, in denen jeweils der Abstand zu einer horizontalen Decke 50 gemessen werden soll, wenn die Person 10 in Seitenlage auf der Matratze 47 liegt. Für diese Abstandsmessung kann wieder das Abstandsmessgerät 12 der Personenvermessungsstation 1 verwendet werden, wobei dieses den jeweils gemessenen Abstand automatisch an die Auswerteeinheit 17 übermittelt. Die jeweilige Hilfsperson kann für diese Abstandsmessungen bspw. wieder die Seitenkontur 11 der Person 10 in der Seitenlage abtasten und die einzelnen Abstände zur Decke 50 ermitteln. Ebenso ist denkbar, mit Hilfe einer entsprechenden, am Abstandsmessgerät 12 angebrachten Hilfskontur, z.B. in Form eines Tasters die Wirbelsäule der liegenden Person 10 abzutasten, um entlang der Wirbelsäule mehrere Abstandswerte gegenüber der Decke 50 zu ermitteln. Im Beispiel ist die Decke 50 durch einen Bestandteil der Einstellüberprüfungsstation 44 gebildet. Es ist jedoch grundsätzlich denkbar, dass auch eine Decke des Raums oder Zimmers verwendet wird, in dem sich die Einstellüberprüfungsstation 44 befindet, solange diese Decke innerhalb der Reichweite des Abstandsmessgeräts 12 liegt und sich horizontal erstreckt. Für die Abstandsmessungen ist der absolute Abstandswert nicht erforderlich, wenn nur die relative Lage der Wirbelsäule zur Horizontalebene ermittelt werden soll.

Die Auswerteeinheit 17 kann nun so ausgestaltet sein, dass sie anhand der Abstände, die den einzelnen Bereichen zugeordnet sind, einen Ist-Verlauf der Wirbelsäule der Person 10 ermitteln kann. Ferner kann die Auswerteeinheit 17 eine Abweichung dieses Ist-Verlaufs von einem Soll-Verlauf ermitteln. Dieser Soll-Verlauf entspricht dabei weitgehend einem geradlinigen Verlauf der Wirbelsäule in einer horizontalen Ebene für die Person 10 in Seitenlage.

Besonders zweckmäßig ist eine Ausführungsform, bei welcher die Auswerteinheit 17 anhand der ermittelten Seitenkontur 11 Einstellparameter für das Liegesystem 54, insbesondere für die Unterfederung 46, generiert. Diese Einstellparameter sind dabei so gewählt, dass sie, wenn das Liegesystem 54 bzw. die Unterfederung 46 mit Hilfe dieser Einstellparameter eingestellt ist, erfahrungsgemäß dazu führen, dass sich für die Wirbelsäule der Person 10, wenn sie in Seitenlage auf der Matratze 47 liegt, der Soll-Verlauf einstellt. Ziel der Einstellung der Unterfederung 46 ist für Seitenschläfer eine horizontale Ausrichtung der Wirbelsäule. Durch Einstellen des Liegesystems 54 bzw. der Unterfederung 46 abhängig von der jeweiligen Seitenkontur 11 lässt sich diese geradlinige Ausrichtung der Wirbelsäule vereinfacht realisieren. Da sich in Verbindung mit dem tatsächlichen Gewicht der jeweiligen Person 10 und in Verbindung mit der jeweils verwendeten Matratze 47 Abweichungen zwischen Ist-Verlauf und Soll-Verlauf einstellen können, kann die mit Hilfe der Personenvermessungsstation 1 ermittelte Einstellung des Liegesystems 54, vorzugsweise der Unterfederung 46, mit Hilfe der Einstellungsüberprüfungsstation 44 überprüft werden. Die Auswerteeinheit 17 kann abhängig vom ermittelten Ist-Verlauf, der in Verbindung mit der Einstellungsüberprüfungsstation 44 ermittelt worden ist, Korrekturparameter für das Liegesystem 54 bzw. die Unterfederung 46 ermitteln, die zu einer Reduzierung und im Idealfall zu einer Eliminierung der Abweichung zwischen Ist-Verlauf und Soll-Verlauf führen, wenn sie am Liegesystem 54 bzw. an der Unterfederung 46 eingestellt werden.

Insbesondere mit Hilfe der vorstehend beschriebenen Personenvermessungsstation 1 lässt sich ein Verfahren zum Vermessen einer Seitenkontur 11 einer Person 10 durchführen, mit dessen Hilfe ein Liegesystem 54, vorzugsweise eine Unterfederung 46, eines Betts 45 eingestellt werden kann. Im Rahmen dieses Verfahrens wird zunächst bei stehender Person mittels eines Abstandsmessgeräts 12 in mehreren vorbestimmten Bereichen 15 entlang einer Seite 16 der jeweiligen Person 10 jeweils ein horizontaler Abstand 14 von der Seite 16 der Person 10 zu einer vertikalen Referenzwand gemessen, die bei der Personenvermessungsstation 1 bevorzugt durch die jeweilige Seitenwand 4, 5 gebildet ist. Bei einer einfacheren Ausführungsform kann diese Referenzwand auch durch eine Gebäudewand gebildet sein, die den Raum bzw. das Zimmer, in dem die Personenvermessungsstation 1 realisiert ist, begrenzt. Denkbar ist dabei auch eine mobile Personenvermessungsstation 1, bei der in einem beliebigen Raum oder Zimmer die Körpervermessung durchgeführt werden kann, solange eine Wand verfügbar ist, die als Referenzwand verwendbar ist. Dabei ist von Bedeutung, dass zum Messen eines solchen Abstands 14 das Abstandsmessgerät 12 mit der Seite 16 der jeweiligen Person 10 in Kontakt gebracht wird und der Abstand 14 zur Seitenwand 4, 5 berührungslos gemessen wird. Hierdurch kann die Messung bei bekleideter Person 10 durchgeführt werden. Gleichzeitig lässt sich die Messung besonders einfach durchführen. Das Abstandsmessgerät 12 übermittelt die gemessenen Abstände 14 automatisch einer Auswerteeinheit 17, die dann aus den gemessenen Abständen 14 die jeweilige Seitenkontur 11 der Person 10 ermittelt. Für die Ermittlung der Seitenkontur 11 kann außerdem die Größe der Person 10 berücksichtigt werden. Die Größenmessung der Person 10 erfolgt in vertikaler Richtung und kann zu Beginn oder zum Schluss der seitlichen oder horizontalen Abstandsmessungen durchgeführt werden. Beispielsweise kann das Abstandsmessgerät 12 hierzu auf den Kopf der Person 10 aufgesetzt und dessen Abstand zur Decke 7 gemessen werden. Aus dem vertikalen Abstand zwischen Kopf und Decke 7 kann die Auswerteeinheit 17 in Verbindung mit dem bekannten Abstand zwischen Boden 8 und Decke 7 die Größe der Person 10 berechnen. Über die Größe der Person 10 kann in Verbindung mit den vorbestimmten Bereichen 15, in denen der Abstand 14 zur Seitenwand 4, 5 gemessen wird, die vertikale Position der einzelnen Messbereiche 16 entlang der Größe der Person 10 bestimmt werden. Darüber hinaus kann hierzu in Verbindung mit der Rückenkonturerfassungseinrichtung 38 auch die vertikale Position eines vorbestimmten, ausschlaggebenden Bereichs 41 der Rückenkontur 40 herangezogen werden. Beispielsweise eignet sich hierfür der Bereich der Lordose. Ebenso ist denkbar, dass einem oder mehreren oder allen horizontal gemessenen Abstandswerten der Seitenkontur 11 jeweils auch eine vertikale Abstandsmessung durchgeführt wird, um dem jeweiligen horizontalen Abstandswert auch eine gemessene vertikale Position zuordnen zu können. Hierdurch lässt sich die jeweilige Seitenkontur 11 genauer bestimmen.

## Patentansprüche

1. Personenvermessungsstation (1) zum Vermessen einer Seitenkontur (11) einer Person (10) zum Einstellen eines Liegesystems (54) eines Betts (45),
- mit einer Rückwand (3), die einen Messraum (2) der Personenvermessungsstation (1) nach hinten begrenzt,
- mit wenigstens einer Seitenwand (4, 5), die den Messraum (2) seitlich begrenzt,
- mit einer Standfläche (6), die den Messraum (2) nach unten begrenzt und auf der sich eine Person (10), deren Seitenkontur (11) vermessen werden soll, in einer Messposition hinstellen kann, in der ein Rücken der Person (10) der Rückwand (3) zugewandt oder davon abgewandt ist und eine Seite (16) der Person (10) der jeweiligen Seitenwand (4, 5) zugewandt ist,
- mit einem Abstandsmessgerät (12) zum Messen eines Abstands (14) von einem vorbestimmten Bereich (15) der Seite (16) der Person (10) zu der zugewandten Seitenwand (4, 5),
- mit einer Auswerteeinheit (17), die mit dem Abstandsmessgerät (12) zur Signalübertragung gekoppelt ist,
- wobei die Personenvermessungsstation (1) als Einheit zur Anordnung in einem Raum oder Zimmer eines Gebäudes ausgestaltet ist,
**dadurch gekennzeichnet,**
- **dass** das Abstandsmessgerät (12) ein hinteres Ende (22) und ein vorderes Ende (23) aufweist,
- **dass** das Abstandsmessgerät (12) zum Messen des jeweiligen Abstands (14) an dem hinteren Ende (22) mit der Seite (16) der Person (10) in Kontakt bringbar ist und an dem vorderen Ende (23) den Abstand (14) berührungslos misst,
- **dass** am vorderen Ende (23) des Abstandsmessgeräts (12) zum Messen des Abstands (14) ein Messstrahl (24) austritt, der auf die Seitenwand (4, 5) auftrifft,
- **dass** das Abstandsmessgerät (12) eine Messeinheit (25) aufweist, die so ausgestaltet ist, dass sie eine am Abstandsmessgerät (12) ankommende Reflexion des Messstrahls (24) detektiert und die Zeit misst, die der Messstrahl (24) vom Abstandsmessgerät (12) zur Seitenwand (4, 5) und zurück benötigt und anhand dieser Zeit den Abstand (14) vom hinteren Ende (22) zur Seitenwand (4, 5) berechnet.

2. Personenvermessungsstation nach Anspruch 1,
**dadurch gekennzeichnet,**
**dass** die Auswerteeinheit (17) so ausgestaltet ist, dass sie anhand der den einzelnen Bereichen (15) zugeordneten Abstände (14) die Seitenkontur (11) der jeweiligen Person (10) ermittelt.

3. Personenvermessungsstation nach einem der vorhergehenden Ansprüche,
**dadurch gekennzeichnet,**
**dass** die jeweilige Seitenwand (4, 5) eine Messzone (27) aufweist, die sich an einem der Rückwand (3) zugewandten hinteren Ende (28) der jeweiligen Seitenwand (4, 5) befindet und deren dem Messraum (2) zugewandte Oberfläche (29) in einer vertikalen Seitenebene (30) liegt, die sich senkrecht zu einer vertikalen Rückseitenebene (31) erstreckt, in der eine dem Messraum (2) zugewandte Oberfläche (32) der Rückwand (3) liegt.

4. Personenvermessungsstation nach Anspruch 3,
**dadurch gekennzeichnet,**
**dass** die jeweilige Seitenwand (4, 5) eine Konvergenzzone (34) aufweist, die nach vorn an die Messzone (27) angrenzt und deren dem Messraum (2) zugewandte Oberfläche (35) in einer vertikalen Konvergenzebene (36) liegt, die sich hinter der Rückwand (3) mit einer vertikalen Mittelebene (33) schneidet, die sich mittig durch die Rückwand (3) und senkrecht zur Rückseitenebene (31) erstreckt.

5. Personenvermessungsstation nach einem der Ansprüche 1 bis 4,
**dadurch gekennzeichnet,**
**dass** in die Rückwand (3) eine Rückenkonturerfassungseinrichtung (38) zum Erfassen einer Rückenkontur (40) einer mit dem Rücken zur Rückwand (3) auf der Standfläche (6) stehenden Person (10) integriert ist.

6. Personenvermessungsstation nach Anspruch 5,
**dadurch gekennzeichnet,**
**dass** die Rückenkonturerfassungseinrichtung (38) eine Vielzahl von horizontal verstellbaren Schiebern (39) aufweist, die vertikal übereinander angeordnet sind und einzeln von einer Ausgangsstellung, in der sie maximal in die Rückwand (3) eingeschoben sind, in eine Messstellung verstellbar sind, in der sie bis zum Kontakt mit dem Rücken der Person (10) aus der Rückwand (3) ausschiebbar sind.

7. Personenvermessungsstation nach Anspruch 6,
**dadurch gekennzeichnet,**
**dass** die Auswerteeinheit (17) so ausgestaltet ist, dass sie zum Vermessen der Rückenkontur (40) zumindest einen Rückenbereich (41) vorgibt, in dem mittels des Abstandsmessgeräts (12) der Abstand eines dem vorgegebenen Rückenbereich (41) zugeordneten Schiebers (39) zu einem die Standfläche (6) aufweisenden horizontalen Boden (8) oder zu einer horizontalen Decke (7) der Personenvermessungsstation (1) gemessen werden soll.

8. Personenvermessungsstation nach Anspruch 6 oder 7,
**dadurch gekennzeichnet,**
**dass** die Rückenkonturerfassungseinrichtung (38) einen Messbalken (55) aufweist, der eine dem Messraum (2) zugewandte Vorderseite (56) aufweist, die gegenüber einer dem Messraum (2) zugewandten Oberfläche (32) der Rückwand (3) nach hinten versetzt angeordnet ist, so dass mit dem Abstandsmessgerät (12) eine Ausfahrlänge wenigstens eines solchen in die Messstellung verstellten Schiebers (39) gegenüber der Vorderseite (56) des Messbalkens (55) messbar ist.

9. Personenvermessungsstation nach Anspruch 8,
**dadurch gekennzeichnet,**
- **dass** der jeweilige Schieber (39) im Bereich einer vertikalen Mittelebene (33) der Personenvermessungsstation (1) eine Aussparung (42) aufweist, die so bemessen ist, dass das Abstandsmessgerät (12) in diese Aussparung (42) einführbar ist,
- **dass** der Messbalken (55) innerhalb der Aussparungen (42) angeordnet ist,
- **dass** insbesondere die Messung der Ausfahrlänge bei in die Aussparung (42) wenigstens eines Schiebers (39) eingesetztem Abstandsmessgerät (12) durch diese Aussparungen (42) zum Messbalken (55) durchführbar ist.

10. Personenvermessungsstation nach einem der Ansprüche 6 bis 9,
**dadurch gekennzeichnet,**
**dass** der jeweilige Schieber (39) im Bereich einer vertikalen Mittelebene (33) der Personenvermessungsstation (1) eine Aussparung (42) aufweist, die so bemessen ist, dass das Abstandsmessgerät (12) in diese Aussparung (42) einführbar ist und insbesondere die Abstandsmessung zum Boden (8) oder zur Decke (7) bei in die Aussparungen (42) mehrerer Schieber (39) eingesetztem Abstandsmessgerät (12) durch diese Aussparungen (42) der Schieber (39) hindurch durchführbar ist.

11. Betteinstelleinrichtung zum Einstellen eines Betts (45) für eine Person (10),
- mit einer Personenvermessungsstation (1) nach einem der Ansprüche 1 bis 10,
- mit einer Einstellungsüberprüfungsstation (44), die ein Bett (45) mit einem Liegesystem (54) aufweist,
- wobei das Liegesystem (54) abhängig von der in der Personenvermessungsstation (1) ermittelten Seitenkontur (11) einstellbar ist, um das Liegesystem (54) an die Seitenkontur (11) der Person (10) anzupassen,
- wobei die Auswerteeinheit (17) der Personenvermessungsstation (1) so ausgestaltet ist, dass sie zum Überprüfen der Einstellung des Liegesystems (54) mehrere Bereiche der Person (10) vorgibt, in denen jeweils der Abstand zu einer horizontalen Decke (50) zu messen ist, wenn die Person (10) in Seitenlage auf dem Bett (45) liegt,
- wobei das Abstandsmessgerät (12) so ausgestaltet ist, dass es für den jeweiligen Bereich den gemessenen Abstand zur Decke (50) automatisch an die Auswerteeinheit (17) übermittelt,
- wobei die Auswerteeinheit (17) so ausgestaltet ist, dass sie anhand der den einzelnen Bereichen zugeordneten Abstände einen Ist-Verlauf der Wirbelsäule der Person (10) und eine Abweichung dieses Ist-Verlaufs von einem Soll-Verlauf ermittelt.

12. Betteinstelleinrichtung nach Anspruch 11,
**dadurch gekennzeichnet,**
**dass** die Auswerteeinheit (17) so ausgestaltet ist, dass sie anhand der ermittelten Seitenkontur (11) Einstellparameter für das Liegesystem (54) ermittelt, die dazu führen sollen, dass sich für die Wirbelsäule der Person (10), wenn sie in Seitenlage auf dem Referenzbett (45) liegt, dessen Liegesystem (54) mit diesen Einstellparametern eingestellt ist, der Soll-Verlauf einstellt.

13. Betteinstelleinrichtung nach Anspruch 11 oder 12,
**dadurch gekennzeichnet,**
**dass** die Auswerteeinheit (17) so ausgestaltet, dass sie abhängig vom ermittelten Ist-Verlauf Korrekturparameter für das Liegesystem (54) ermittelt, die zu einer Reduzierung der Soll-Ist-Differenz führen.

14. Verfahren zum Vermessen einer Seitenkontur (11) einer Person (10) zum Einstellen eines Liegesystems (54) eines Betts (45) mit einer Personenvermessungsstation (1) nach einem der Ansprüche 1 bis 10,
- bei dem bei stehender Person (10) mittels des Abstandsmessgeräts (12) in mehreren Bereichen (15) entlang einer Seite (16) der jeweiligen Person (10) jeweils ein Abstand (14) von der Seite (16) der Person (10) zu einer Seitenwand (4, 5) gemessen wird,
- bei dem zum Messen eines solchen Abstands (14) das Abstandsmessgerät (12) mit der Seite (16) der jeweiligen Person (10) in Kontakt gebracht wird und der Abstand (14) zur Seitenwand (4, 5) berührungslos gemessen wird,
- bei dem die mit dem Abstandsmessgerät (12) gemessenen Abstände (14) automatisch einer Auswerteeinheit (17) übermittelt werden, die aus den Abständen (14) die Seitenkontur (11) ermittelt.

## Claims

1. Person measuring station (1) for measuring a side contour (11) of a person (10) for adjusting a lying system (54) of a bed (45), comprising
- a rear wall (3), which delimits a measuring space (2) of the person measuring station (1) at the rear,
- at least one side wall (4, 5), which delimits the measuring space (2) at the side,
- a standing surface (6), which delimits the measuring space (2) at the bottom, and on which a person (10), whose side contour (11) is to be measured, can position himself in a measuring position, in which the back of the person (10) faces the rear wall (3) or faces away from the latter and a side (16) of the person (10) faces the respective side wall (4, 5),
- a distance measuring device (12) for measuring a distance (14) from a prespecified area (15) of the side (16) of the person (10) to the facing side wall (4, 5),
- an evaluating unit (17), which is coupled to the distance measuring device (12) for transmitting the signal,
- wherein the person measuring station (1) is configured as a unit for placing in a space or room of a building,
**characterised in that**
- the distance measuring device (12) has a rear end (22) and a front end (23),
- the distance measuring device (12) for measuring the respective distance (14) at the rear end (22) can be brought into contact with the side (16) of the person (10) and at the front end (23) measures the distance (14) contactlessly,
- at the front end (23) of the distance measuring device (12) for measuring the distance (14) a measuring beam (24) is emitted which impinges on the side wall (4, 5),
- the distance measuring device (12) has a measuring unit (25) which is configured so that it detects a reflection of the measuring beam (24) arriving at the distance measuring device (12) and measures the time that the measuring beam (24) needs from the distance measuring device (12) to the side wall (4, 5) and back and with reference to this time calculates the distance (14) from the rear end (22) to the side wall (4, 5).

2. Person measuring station according to claim 1,
**characterised in that**
the evaluating unit (17) is configured so that it determines the side contour (11) of the respective person (10) with reference to the distances (14) assigned to the individual areas (15).

3. Person measuring station according to any of the preceding claims,
**characterised in that**
the respective side wall (4, 5) has a measuring area (27) which is located on a rear end (28) of the respective side wall (4, 5) facing the rear wall (3) and whose surface (29) facing the measuring space (2) is in a vertical side plane (30), which extends perpendicular to a vertical rear plane (31), in which a surface (32) of the rear wall (3) facing the measuring space (2) lies.

4. Person measuring station according to claim 3,
**characterised in that**
the respective side wall (4, 5) has a convergence area (34), which to the front adjoins the measuring area (27) and whose surface (35) facing the measuring space (2) is in a vertical convergence plane (36), which intersects behind the rear wall (3) with a vertical central plane (33) which extends centrally through the rear wall (3) and perpendicular to the rear plane (31).

5. Person measuring station according to any of claims 1 to 4,
**characterised in that**
a rear contour detection device (38) is integrated into the rear wall (3) for detecting a rear contour (40) of a person (10) standing on the standing surface (6) with their back to the rear wall (3).

6. Person measuring station according to claim 5,
**characterised in that**
the rear contour detection device (38) comprises a plurality of horizontally adjustable slides (39) which are arranged vertically above one another and can be adjusted individually from a starting position in which they are inserted to the maximum into the rear wall (3) into a measuring position, in which they can be pushed out of the rear wall (3) until they contact the back of the person (10).

7. Person measuring station according to claim 6,
**characterised in that**
the evaluating unit (17) is configured so that it predefines at least one back area (41) for measuring the rear contour (40), in which back area by means of the distance measuring device (12) the distance of a slide (39) assigned to the predefined back area (41) to a horizontal base (8) having the standing surface (6) or a horizontal cover (7) of the person measuring station (1) is to be measured.

8. Person measuring station according to claim 6 or 7,
**characterised in that**
the rear contour detection device (38) has a measuring bar (55), which has a front side (56) facing the measuring space (2), which front side is arranged offset to the rear relative to a surface (32) of the rear wall (3) facing the measuring space (2) so that by means of the distance measuring device (12) an extension length of at least one such slide (39) adjusted into the measurement position can be measured relative to the front side (56) of the measuring bar (55).

9. Person measuring station according to claim 8,
**characterised in that**
- the respective slide (39) in the area of a vertical central plane (33) of the person measuring station (1) has a recess (42) which is dimensioned so that the distance measuring device (12) can be inserted into said recess (42),
- the measuring bar (55) is arranged inside the recesses (42),
- in particular the measurement of the extension length with a distance measuring device (12) inserted into the recess (42) of at least one slide (39) can be performed through said recesses (42) to the measuring bar (55).

10. Person measuring station according to any of claims 6 to 9,
**characterised in that**
the respective slide (39) in the area of a vertical central plane (33) of the person measuring station (1) has a recess (42) which is dimensioned so that the distance measuring device (12) can be inserted into said recess (42) and in particular the distance measurement to the base (8) or to the cover (7) with a distance measuring device (12) inserted into the recesses (42) of a plurality of slides (39) can be performed through said recesses (42) of the slide (39).

11. Bed adjusting device for adjusting a bed (45) for a person (10), comprising
- a person measuring station (1) according to any of claims 1 to 10,
- an adjustment checking station (44) which has a bed (45) with a lying system (54),
- wherein the lying system (54) can be adjusted depending on the side contour (11) determined in the person measuring station (1), in order to adjust the lying system (54) to the side contour (11) of the person (10),
- wherein the evaluating unit (17) of the person measuring station (1) is configured so that it predefines a plurality of areas of the person (10) to check the adjustment of the lying system (54), in which respectively the distance to a horizontal cover (50) should be measured when the person (10) is lying on their side on the bed (45),
- wherein the distance measuring device (12) is configured so that for the respective area it automatically transmits the measured distance to the cover (50) to the evaluating unit (17),
- wherein the evaluating unit (17) is configured so that with reference to the distances assigned to the individual areas it determines an actual course of the spine of the person (10) and a deviation of said actual course from a reference course.

12. Bed adjusting device according to claim 11,
**characterised in that**
the evaluating unit (17) is configured so that with reference to the determined side contour (11) it determines adjusting parameters for the lying system (54) which should mean that the intended course is adjusted for the spine of the person (10), when laying on their side on the reference bed (45), the lying system (54) of which has been adjusted by said adjusting parameters.

13. Bed adjusting device according to claim 11 or 12,
**characterised in that**
the evaluating unit (17) is configured so that depending on the determined actual course it determines correction parameters for the lying system (54) which lead to a reduction of the difference between the intended and actual position.

14. Method for measuring a side contour (11) of a person (10) for adjusting a lying system (54) of a bed (45) with a person measuring station (1) according to any of claims 1 to 10,
- in which with a standing person (10) a distance (14) from the side (16) of the person (10) to a side wall (4, 5) is measured by means of the distance measuring device (12) in several areas (15) along one side (16) of the respective person (10) respectively,
- in which for measuring such a distance (14) the distance measuring device (12) is brought into contact with the side (16) of the respective person (10) and the distance (14) to the side wall (4, 5) is measured contactlessly,
- in which the distances (14) measured by the distance measuring device (12) are transmitted automatically to an evaluating unit (17), which from the distances (14) determines the side contour (11).

## Revendications

1. Poste de prise de mesures de personne (1) destiné à mesurer un contour de côté (11) d'une personne (10) dans le but de régler un système de couchage (54) d'un lit (45),
- avec une paroi arrière (3) qui délimite vers l'arrière un espace de mesure (2) du poste de prise de mesures de personne (1),
- avec au moins une paroi latérale (4, 5), qui délimite latéralement l'espace de mesure (2),
- avec une surface de base (6), qui délimite vers le bas l'espace de mesure (2) et sur laquelle une personne (10) dont le contour de côté (11) doit être mesuré peut se placer dans une position de mesure dans laquelle un dos de la personne (10) est tourné vers la paroi arrière (3) ou à l'opposé de celle-ci et un côté (16) de la personne (10) est tourné vers la paroi latérale (4, 5) respective,
- avec un appareil de mesure de distance (12) destiné à mesurer une distance (14) entre une zone prédéterminée (15) du côté (16) de la personne (10) et la paroi latérale (4, 5) à proximité,
- avec une unité d'évaluation (17), qui est couplée à l'appareil de mesure de distance (12) en vue de la transmission de signaux,
- dans lequel le poste de prise de mesures de personne (1) est réalisé sous la forme d'une unité destinée à être placée dans un espace ou une pièce d'un bâtiment, **caractérisé en ce que**
- l'appareil de mesure de distance (12) comporte une extrémité arrière (22) et une extrémité avant (23),
- pour mesurer la distance (14) respective, l'appareil de mesure de distance (12) peut être mis en contact au niveau de l'extrémité arrière (22) avec le côté (16) de la personne (10) et mesure la distance (14) sans contact au niveau de l'extrémité avant (23),
- pour mesurer la distance (14), au niveau de l'extrémité avant (23) de l'appareil de mesure de distance (12) sort un faisceau de mesure (24) qui arrive sur la paroi latérale (4, 5),
- l'appareil de mesure de distance (12) comporte une unité de mesure (25) qui est réalisée de telle sorte qu'elle détecte une réflexion, arrivant au niveau de l'appareil de mesure de distance (12), du faisceau de mesure (24) et qu'elle mesure le temps qui est nécessaire au faisceau de mesure (24) pour aller de l'appareil de mesure de distance (12) à la paroi latérale (4, 5) et revenir et qu'elle calcule à l'aide de ce temps la distance (14) entre l'extrémité arrière (22) et la paroi latérale (4, 5).

2. Poste de prise de mesures de personne selon la revendication 1,
**caractérisé en ce**
**que** l'unité d'évaluation (17) est réalisée de telle sorte qu'elle détermine à l'aide des distances (14) associées aux zones (15) individuelles le contour de côté (11) de la personne (10) respective.

3. Poste de prise de mesures de personne selon l'une quelconque des revendications précédentes,
**caractérisé en ce**
**que** la paroi latérale (4, 5) respective comporte une zone de mesure (27) qui se trouve au niveau d'une extrémité arrière (28), proche de la paroi arrière (3), de la paroi latérale (4, 5) respective et dont la surface (29) proche de l'espace de mesure (2) se trouve dans un plan latéral vertical (30) qui s'étend perpendiculairement à un plan arrière vertical (31) dans lequel se trouve une surface (32), proche de l'espace de mesure (2), de la paroi arrière (3).

4. Poste de prise de mesures de personne selon la revendication 3,
**caractérisé en ce**
**que** la paroi latérale (4, 5) respective comporte une zone de convergence (34) qui est adjacente vers l'avant à la zone de mesure (27) et dont la surface (35) proche de l'espace de mesure (2) se trouve dans un plan de convergence vertical (36) qui coupe, derrière la paroi arrière (3), un plan médian vertical (33) qui s'étend au milieu à travers la paroi arrière (3) et perpendiculairement au plan arrière (31).

5. Poste de prise de mesures de personne selon l'une quelconque des revendications 1 à 4,
**caractérisé en ce**
**que** dans la paroi arrière (3) est intégré un dispositif de détection de contour de dos (38) en vue de la détection d'un contour de dos (40) d'une personne (10) debout sur la surface de base (6) avec le dos vers la paroi arrière (3).

6. Poste de prise de mesures de personne selon la revendication 5,
**caractérisé en ce**
**que** le dispositif de détection de contour de dos (38) comporte une multiplicité de coulisseaux (39) déplaçables horizontalement qui sont agencés verticalement les uns au-dessus des autres et qui peuvent être déplacés individuellement d'une position de départ dans laquelle ils sont glissés au maximum dans la paroi arrière (3) à une position de mesure dans laquelle ils sont glissés hors de la paroi arrière (3) jusqu'au contact avec le dos de la personne (10).

7. Poste de prise de mesures de personne selon la revendication 6,
**caractérisé en ce**
**que** l'unité d'évaluation (17) est réalisée de telle sorte que, pour mesurer le contour de dos (40), elle prescrit au moins une zone de dos (41) dans laquelle doit être mesurée au moyen de l'appareil de mesure de distance (12) la distance entre un coulisseau (39) associé à la zone de dos (41) prescrite et un sol horizontal (8) comportant la surface de base (6) ou un plafond horizontal (7) du poste de prise de mesures de personne (1).

8. Poste de prise de mesures de personne selon la revendication 6 ou 7,
**caractérisé en ce**
**que** le dispositif de détection de contour de dos (38) comporte une barre de mesure (55) qui comporte une face avant (56) qui est proche de l'espace de mesure (2) et qui est agencée de manière décalée vers l'arrière par rapport à une surface (32), proche de l'espace de mesure (2), de la paroi arrière (3), de sorte qu'il est possible de mesurer avec l'appareil de mesure de distance (12) une longueur de déploiement d'au moins un coulisseau (39), déplacé dans la position de mesure, par rapport à la face avant (56) de la barre de mesure (55).

9. Poste de prise de mesures de personne selon la revendication 8,
**caractérisé en ce que**
- le coulisseau (39) respectif comporte dans la zone d'un plan médian vertical (33) du poste de prise de mesures de personne (1) un évidement (42) qui est dimensionné de telle sorte que l'appareil de mesure de distance (12) peut être introduit dans cet évidement (42),
- la barre de mesure (55) est agencée à l'intérieur des évidements (42),
- la mesure de la longueur de déploiement peut être effectuée en particulier, lorsque l'appareil de mesure de distance (12) est placé dans l'évidement (42) d'au moins un coulisseau (39), à travers ces évidements (42) vers la barre de mesure (45).

10. Poste de prise de mesures de personne selon l'une quelconque des revendications 6 à 9,
**caractérisé en ce**
**que** le coulisseau respectif (39) comporte dans la zone d'un plan médian vertical (33) du poste de prise de mesures de personne (1) un évidement (42) qui est dimensionné de telle sorte que l'appareil de mesure de distance (12) peut être introduit dans cet évidement (42) et **en ce que** la mesure de distance au sol (8) ou au plafond (7) peut être effectuée, lorsque l'appareil de mesure de distance (12) est placé dans les évidements (42) de plusieurs coulisseaux (39), à travers ces évidements (42) des coulisseaux (39).

11. Dispositif de réglage de lit destiné à régler un lit (45) pour une personne (10),
- avec un poste de prise de mesures de personne (1) selon l'une quelconque des revendications 1 à 10,
- avec un poste de vérification de réglage (44), qui comporte un lit (45) avec un système de couchage (54),
- dans lequel le système de couchage (54) peut être réglé en fonction du contour de côté (11) déterminé dans le poste de prise de mesures de personne (1) afin d'adapter le système de couchage (54) au contour de côté (11) de la personne (10),
- dans lequel l'unité d'évaluation (17) du poste de prise de mesures de personne (1) est réalisée de telle sorte que, pour vérifier le réglage du système de couchage (54), elle prescrit plusieurs zones de la personne (10) dans lesquelles à chaque fois la distance à un plafond horizontal (50) est à mesurer lorsque la personne (10) se trouve en position couchée sur le côté sur le lit (45),
- dans lequel l'appareil de mesure de distance (12) est réalisé de telle sorte qu'il transmet automatiquement à l'unité d'évaluation (17) la distance au plafond (50) mesurée pour la zone respective,
- dans lequel l'unité d'évaluation (17) est réalisée de telle sorte que, à l'aide des distances associées aux zones individuelles, elle détermine une allure réelle de la colonne vertébrale de la personne (10) et un écart de cette allure réelle à une allure de consigne.

12. Dispositif de réglage de lit selon la revendication 11,
**caractérisé en ce**
**que** l'unité d'évaluation (17) est réalisée de telle sorte que, à l'aide du contour de côté (11) déterminé, elle détermine des paramètres de réglage pour le système de couchage (54) qui doivent conduire à ce que l'allure de consigne est atteinte pour la colonne vertébrale de la personne (10) lorsqu'elle se trouve en position couchée sur le côté sur le lit de référence (45) dont le système de couchage (54) est réglé avec ces paramètres de réglage.

13. Dispositif de réglage de lit selon la revendication 11 ou 12,
**caractérisé en ce**
**que** l'unité d'évaluation (17) est réalisée de telle sorte que, à l'aide de l'allure réelle déterminée, elle détermine des paramètres de correction pour le système de couchage (54) qui conduisent à une réduction de la différence entre allure réelle et allure de consigne.

14. Procédé destiné à prendre les mesures d'un contour de côté (11) d'une personne (10) afin de régler un système de couchage (54) d'un lit (45) avec un poste de prise de mesures de personne (1) selon l'une quelconque des revendications 1 à 10,
- selon lequel, alors que la personne (10) est debout, on mesure à chaque fois au moyen de l'appareil de mesure de distance (12), dans plusieurs zones (15) le long d'un côté (16) de la personne respective (10), une distance (14) du côté (16) de la personne (10) à une paroi latérale (4, 5),
- selon lequel, pour mesurer une telle distance (14), on met l'appareil de mesure de distance (12) en contact avec le côté (16) de la personne respective (10) et on mesure ainsi sans contact la distance (14) à la paroi latérale (4, 5),
- selon lequel on transmet automatiquement les distances (14) mesurées avec l'appareil de mesure de distance (12) à une unité d'évaluation (17) qui détermine le contour de côté (11) à partir des distances (14).
